(19) Europäisches Patentamt / European Patent Office / Office européen des brevets

(11) **EP 3 921 626 B1**

(12) **EUROPEAN PATENT SPECIFICATION**

(45) Date of publication and mention
of the grant of the patent:
**08.01.2025 Bulletin 2025/02**

(21) Application number: **19751456.5**

(22) Date of filing: **07.02.2019**

(51) International Patent Classification (IPC):
*G01N 15/1429* *(2024.01)*     *G01N 15/1434* *(2024.01)*
*G01N 21/47* *(2006.01)*     *G01N 33/48* *(2006.01)*
*G01N 21/51* *(2006.01)*

(52) Cooperative Patent Classification (CPC):
**G01N 21/4788; G01N 15/1429; G01N 15/1434;
G01N 21/51;** G01N 2015/1006

(86) International application number:
**PCT/IL2019/050154**

(87) International publication number:
**WO 2019/155470 (15.08.2019 Gazette 2019/33)**

(54) **APPARTATUS AND METHODS FOR REAL-TIME CELL MONITORING**

VORRICHTUNG UND VERFAHREN ZUR ECHTZEITZELLENÜBERWACHUNG

APPAREILS ET PROCÉDÉS DE SURVEILLANCE DE CELLULES EN TEMPS RÉEL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**15.12.2021 Bulletin 2021/50**

(73) Proprietor: **PHENOFAST LTD**
**35083409 Haifa (IL)**

(72) Inventors:
• **SARIEL, Aviram**
**4593000 Ramot Hashavim (IL)**
• **BEN DOV, Nadav**
**3082500 Ein Ayala (IL)**

(74) Representative: **Boult Wade Tennant LLP**
**Salisbury Square House**
**8 Salisbury Square**
**London EC4Y 8AP (GB)**

(56) References cited:
WO-A1-2016/142878     US-A- 4 876 208
US-A1- 2009 180 932     US-A1- 2011 059 538
US-A1- 2016 061 822     US-A1- 2016 061 822

**Description**

**CROSS REFERENCE TO RELATED APPLICATIONS**

This application claims the benefit of priority of U.S. Provisional Patent Application No. 62/627,797, filed February 8, 2018.

**FIELD OF INVENTION**

The present invention is in the field of cell monitoring.

**BACKGROUND OF THE INVENTION**

The Center for Disease Control (CDC) estimates that in the United States, more than two million people are sickened every year with antibiotic-resistant infections, with at least 23,000 dying as a result. Further it emphasizes the importance of developing improved diagnostic tools - which are needed to determine resistance profiles to inform the appropriate use of drugs. Such tools should also reduce the widespread overuse and misuse of the current antibiotic arsenal.

The most important outcome of any antimicrobial susceptibility testing (AST) is the rapid and reliable prediction of a successful antimicrobial agent in the treatment of infection. Limited numbers of methods for AST of medically important microorganisms have survived the maturation of modern diagnostic clinical microbiology.

*In vitro* AST can be performed using a variety of phenotypic formats, the most common being disk diffusion, agar dilution, broth microdilution and a concentration gradient test. At present, expensive nucleic acid-based AST methods cannot detect all resistance markers, and thus have not been widely adopted. Next generation whole genome sequencing is confounded by the ever-elevating degree of genetic heterogeneity, a persistent problem expected to frustrate the genomic approach. There are hundreds of β-lactamases and numerous mutations, acquisitions and expression mechanisms that result in fluoroquinolone, aminoglycoside and macrolide resistance; too many to be easily detected by current molecular techniques. Thus, it is likely that phenotypic measures of the level of susceptibility of bacterial isolates to antimicrobial agents will continue to be clinically relevant for years to come.

Currently, AST is typically accomplished using either classical manual methods or growth-dependent automated systems. The major limitations of these methods include the requirement for relatively large numbers of viable starting organisms, complicated pre-analytical processing, limited organism spectrum, analytical variability, lengthy time to results and cost. The standard diagnostic techniques have not significantly changed during the past 20 years, though these techniques have been adapted into automated instruments and optimized. Still, the complete analysis time has typically improved by only 10-12 hours, which still translates into two working days to provide AST analysis, at best. The laboratory automatization is limited (due to cost and infrastructure) to consolidated central labs, such as large hospital and regional service labs. Local clinics, small hospitals, senior residence, day care facilities and other medical institutions either perform the classic labor-intensive AST or transport patient's samples to centralized locations for automated processing. This intensifies the problem of AST cost and duration, adding the cost and time consumed by transportation. Thus, in the absence of rapid definitive microbiological diagnosis at the point of care (POC), physicians frequently initiate empirical broad-spectrum antibiotic treatment. This leads in many cases to significantly increased risk of clinical adverse outcome, increased mortality, and has contributed to the emergence of resistant pathogens.

An example, for the use of broadband illumination in a system for detecting target elements such as bacteria in a host analyte, is given in U.S. Patent Application Publication No. 2016/0061822A1. The system for detecting target elements such as bacteria in a host analyte, comprising a substrate with an ordered array of wells having diameters to fit the size of the targets. The substrate may be a periodic macro-PSi array structure (MPSiAS) illuminated with a broadband source. The reflected light spectrum diffracted from the substrate is optically analyzed to provide the effective optical depth of the wells. Fast Fourier Transform analysis may be used for the optical analysis. Entry of target elements into wells is detected by the change in the effective optical depths of the wells. Micro-organisms as large as bacteria and viruses having dimensions comparable with the wavelength of the illumination can thus be detected. Wells with an inner section impenetrable by the target cells enables compensation for environmental changes. The detection may be performed in real time, such that production line bacterial monitoring may be achieved.

Another example, for the use of broadband illumination in a method for determining a phenotype of cells, is given in International Patent Application Publication No. WO2016142878A1. The methods are based measuring a refractive index of said cells based on a diffraction pattern received from a diffraction grating having a plurality of compartments having lateral dimensions such that said cells can fit therein. The method includes (a) incubating for a predetermined time the biological sample comprising the cells on a diffraction grating containing an ordered array of compartments having lateral dimensions such that the cells can fit therein; (b) continuously detecting over the predetermined time illumination diffracted from the diffraction grating comprising the cells upon illumination of the surface with illumination over a range of wavelengths and generating therefrom an output spectrum signal; and (c) determining from the output spectrum signal a time-dependence change of an effective optical thickness (EOT) of the compartments incubated with the biological sample, the time-dependence being indicative of the phenotype of the cells in the biological sample.

There is an urgent, recognizable, need for development of new automated instruments that can provide faster results and also save money by virtue of lower reagent costs and reduced labor requirements.

## SUMMARY OF THE INVENTION

The present invention provides systems for measuring cellular growth comprising a diffraction grating, a light source configured to illuminate at a limited waveband, an optical detection system configured to detect zero and non-zero diffraction orders of light, and a signal processing unit adapted to analyze zero and non-zero diffraction orders to determine time-dependent changes in effective optical depth, as well as methods of use thereof.

According to a first aspect, there is provided a system as disclosed in claim 1.

According to another aspect, there is provided a method according to claim 5 for real-time detection of cell replication or growth. Advantageous further developments of the invention are set forth in the dependent claims.

## BRIEF DESCRIPTION OF THE DRAWINGS

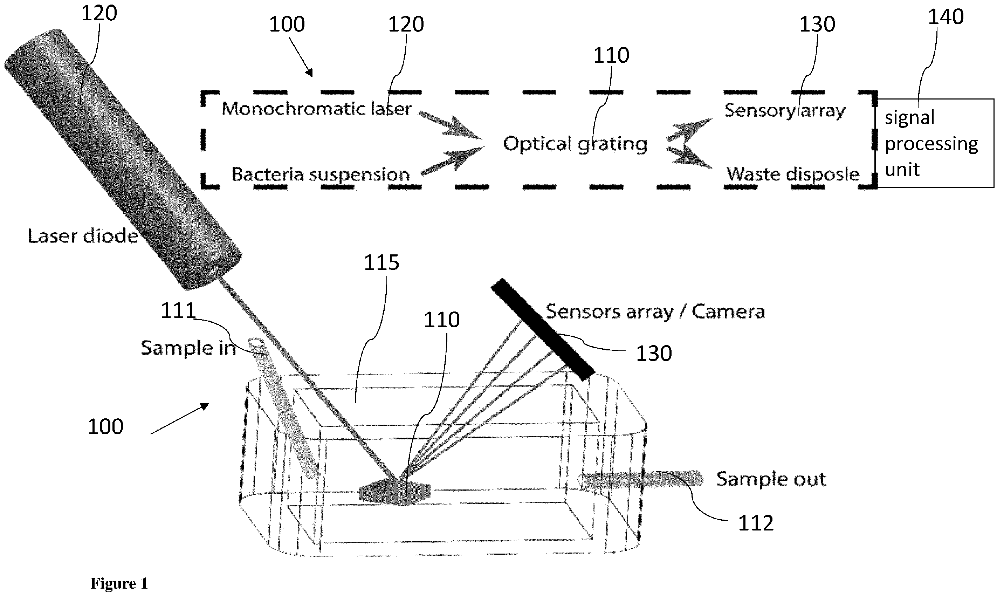

**Figure 1:** A schematic of the system 100 of the invention.

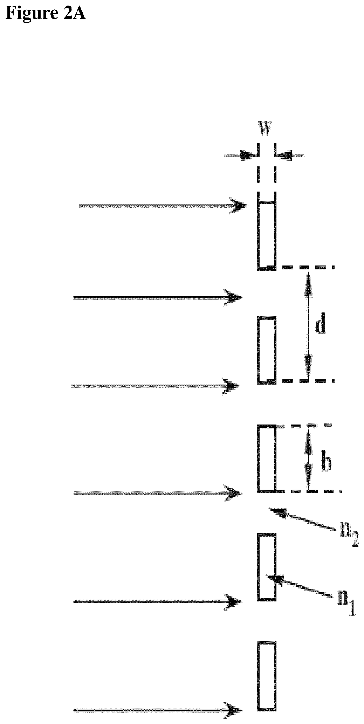

**Figures 2A-2D:** (**2A**) Diagram schematic of the structure of the optical grating. The refraction indexes of the two kinds of materials are noted by $n_1$ and $n_2$, respectively. **d** and **b** are the grating dimensions, **w** is the grating depth and **m** is the diffraction order. **L** is the total width of all the gratings. (**2B**) Photograph of light passing through an optical grating produce a diffraction pattern. (**2C**) Schematic of light intensity ($I_\theta$) of a diffraction pattern, with conventional notation. (**2D**) Photograph of a partial, quarter view of a diffraction distribution. The arrow points to the Zero-order diffraction, seen adjacent to the direct reflection of the laser (650 nm). Grating dimensions are: b = 3 $\mu$m, d = 4 $\mu$m, w = 3 $\mu$m.

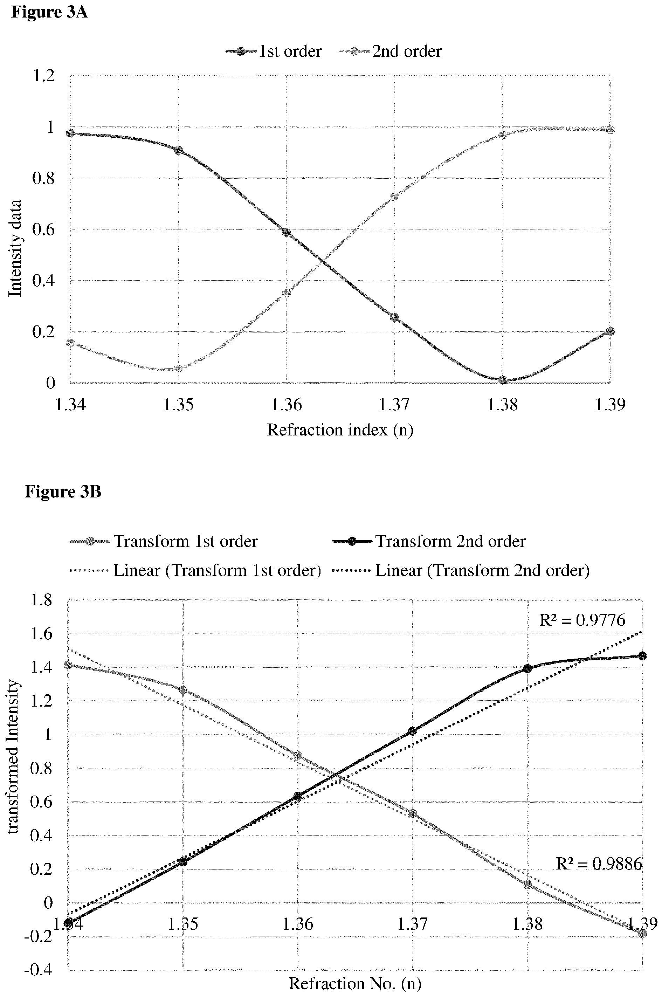

**Figures 3A-3B:** Line graphs of (**3A**) the intensities of two adjacent diffraction orders (odd first order and even second order) as plotted against the solution refraction index and (**3B**) the transformed data using a correction factor (p) of p = -0.5 for the grating used.

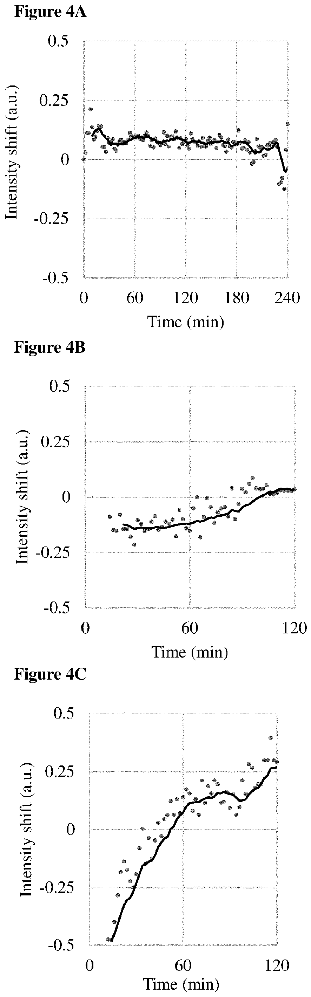

**Figures 4A-4C:** Scatter plots, with best fit lines of optical output measured by intensity shift as a function of time, performed on a series of 6 uncoated grating units in parallel inoculated with (**4A**) 0, (**4B**) 10^5, and (**4C**) 10^7 E. coli cells.

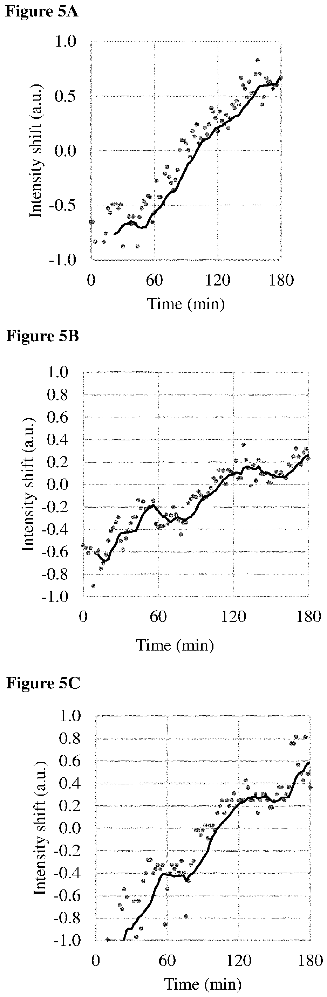

**Figures 5A-5D:** Scatter plots, with best fit lines of optical output measured by intensity shift as a function of time, performed on a series of 6 semi-stable sucrose coated grating units in parallel inoculated with (**5A**) 10^3, (**5B**) 10^4, and (**5C**) 10^5 E. coli cells. (**5D**) Line graphs of relative light intensity between diffraction orders, which indicates cell growth, of three starting concentrations of bacteria seeded on sensor chips (diffraction gratings).

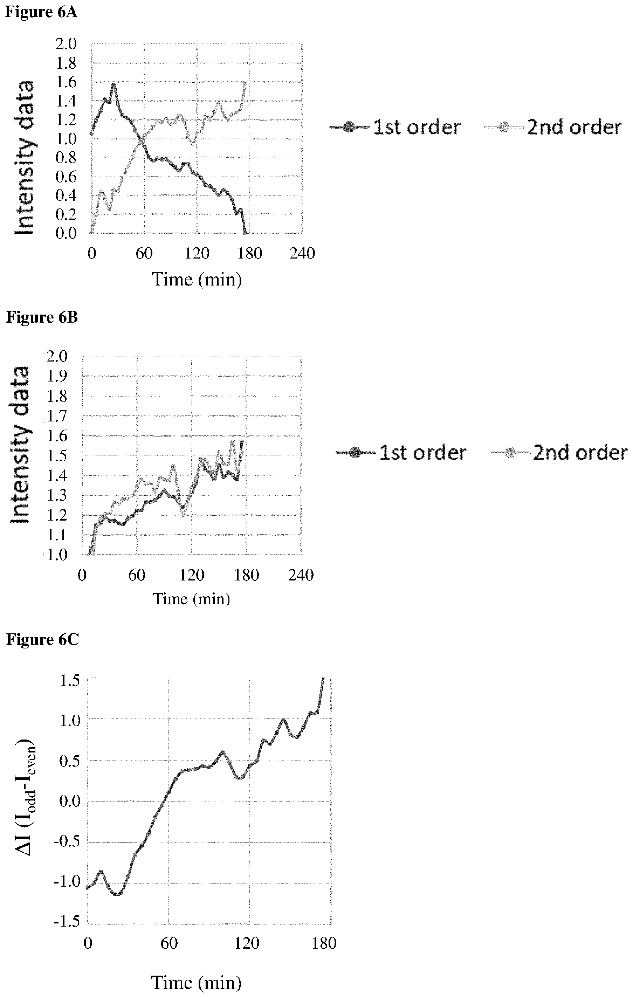

**Figures 6A-6D:** Line graph of (**6A-B**) of first and second order diffraction from optical grating inoculated with (**6A**) bacterial cells and (**6B**) without cells, and of (**6C-D**) the intensity shift resulting from (**6C**) the cell growth and (**6D**) the lack thereof when no cells are present.

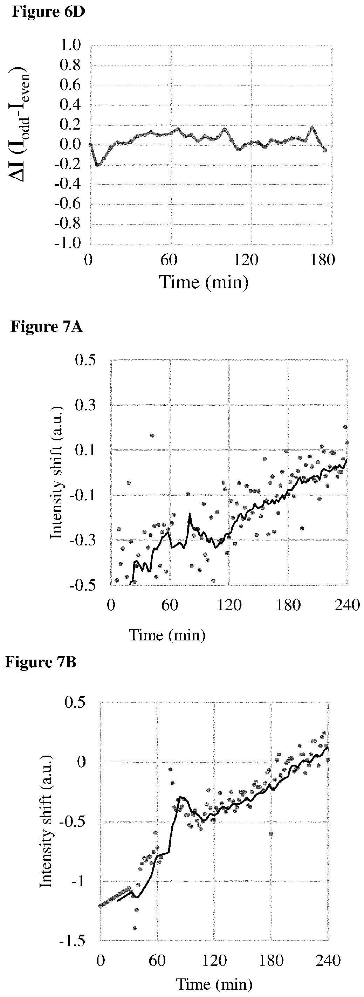

**Figures 7A-7E:** (**7A-7D**) Scatter plots, with best fit lines of optical output measured by intensity shift as a function of time, performed on a series of 6 semi-stable sucrose coated grating units in parallel inoculated with E. coli cells and treated an hour later with (**7A**) 0 $\mu$g, (**7B**) 2.5 $\mu$g, (**7C**) 5.0 $\mu$g, and (**7D**) 7.5 $\mu$g of G418. (**7E**) A photograph of the classical serial dilution method for determining the MIC of G418 for treating E. coli.

## DETAILED DESCRIPTION OF THE INVENTION

The present invention, in some embodiments, provides systems for measuring cellular growth, comprising a diffraction grating, a light source configured to illuminate at a limited waveband, an optical detection system configured to detect zero and non-zero diffraction orders of light, and a signal processing unit adapted to analyze zero and non-zero diffraction orders to determine time-dependent changes in effective optical depth. The present invention further concerns methods for use of these systems for real-time detection of cell growth or deterioration, detection of a cell in a sample, such as a water sample, determining the identity of a bacterial cell and for determining the sensitivity of infectious microorganisms to therapeutic agents, such as may be used in the treatment of an infection in a subject in need thereof.

By a first aspect, there is provided a system, the system as defined in claim 1.

In some embodiments, the system is for measuring growth of a cell. In some embodiments, the system is for measuring changes in an amount of biomaterial or cellular material. In some embodiments, the system is for use in real-

time detection. In some embodiments, the system is for use in real-time detection of cell replication. In some embodiments, the system is for use in real-time measuring of cell growth. In some embodiments, the system is for use in real-time detection of changes in an amount of biomaterial or cellular material.

In some embodiments, the signal processing unit is configured to convert the time-dependent changes in effective optical depth into measures of cellular growth, biomaterial, cellular material and/or cellular replication. Each possibility represents a separate embodiment of the invention. In some embodiments, the signal processing unit determines from the changes in effective optical depth the growth of a cell in the grating. In some embodiments, the changes in optical depth are referred to as a shift. In some embodiments, the shift is a relative change in diffraction intensity. In some embodiments, a decrease in effective optical depth indicates an increase in cell growth, biomaterial or cellular replication. In some embodiments, a large positive shift indicates an increase in cell growth, biomaterial or cellular replication. In some embodiments, the shift is at least 0.02, 0.021, 0.022, 0.023, 0.024, 0.025, 0.026, 0.027, 0.028, 0.029, 0.03, 0.031, 0.032, 0.033, 0.034, or 0.035% increase in diffraction intensity. Each possibility represents a separate embodiment of the invention.

By another aspect, there is provided a method as defined by claim 5 for real-time detection of cell growth or replication.

In some embodiments, the method is for measuring changes in an amount of biomaterial or cellular material. In some embodiments, the method is for real-time detection. In some embodiments, the method is for real-time detection of changes in an amount of biomaterial or cellular material. In some embodiments, the cell is a cell of a microorganism.

By another aspect not forming part of present invention, there is provided a method of selecting a therapeutic agent for treating growth of a cell, the method comprising:

> a. inoculating at least one cell into a plurality of gratings of a system of the invention;
> b. administering at least one potential therapeutic agent to at least one diffraction grating from the plurality of diffraction gratings comprising the at least one cell;
> c. illuminating the diffraction gratings comprising the at least one cell with the light beam;
> d. detecting zero and non-zero diffraction orders of the light beam from the illuminated diffraction gratings;
> e. processing the diffraction orders' intensity over time to determine changes in effective optical depth of each diffraction grating comprising at least one cell and therefrom determining a change in amount of cellular material in each diffraction grating comprising the at least one cell over time; and
> f. selecting a therapeutic agent that resulted in arrested growth or deterioration of cellular material as compared to a control;

thereby selecting a therapeutic agent for treatment of growth of the cell.

By another aspect not forming part of present invention, there is provided a method of determining the presence of a cell in a sample, the method comprising:

> a. administering the sample into a diffraction grating of a system of the invention;
> b. illuminating the diffraction grating comprising the sample with the light beam;
> c. detecting zero and non-zero diffraction orders of the light beam from the illuminated diffraction grating comprising the sample; and
> d. processing the diffraction orders' intensity over time to determine changes in effective optical depth of the diffraction grating comprising the sample and therefrom determining a change in amount of cellular material in the diffraction grating comprising the sample over time; wherein an increase in cellular material indicates the presence of the cell in the sample;

thereby determining the presence of the cell in the sample.

By another aspect not forming part of present invention, there is provided a method of determining the identity of a cell in a sample, the method comprising:

> a. administering the sample into a plurality of diffraction grating of a system of the invention wherein said plurality of diffraction gratings comprise at least two different selective growth media in different gratings;
> b. illuminating the diffraction gratings comprising the sample with the light beam;
> c. detecting zero and non-zero diffraction orders of the light beam from the illuminated diffraction gratings comprising the sample; and
> d. processing the diffraction orders' intensity over time to determine changes in effective optical depth of the diffraction grating comprising the sample and therefrom determining a change in amount of cellular material in the diffraction grating comprising the sample over time; wherein an increase in cellular material in a particular selective growth media indicates the identity of the cell;

thereby determining the identity of the cell in the sample.

In some embodiments, the cell is a prokaryotic or a eukaryotic cell. In some embodiments, the cell is a prokaryotic cell. In some embodiments, the cell is a eukaryotic cell. In some embodiments, the cell is a microorganism. In some embodiments, the cell is a cell of a microorganism. In some embodiments, the microorganism is an infectious microorganism. In some embodiments, the cell is a bacterial cell. In some embodiments, the cell is a fungal cell. In some embodiments, the microorganism is a bacterium or a fungus. In some embodiments, the microorganism is a bacterium. In some embodiments, the microorganism is a fungus. In some embodiments, the microorganism is a parasite. In some embodiments, the microorganism is selected from a bacterium, a fungus and a parasite. In some embodiments, the microorganism is from a subject in need of treatment for infection by the microorganism. In some embodiments, the cell is a mammalian cell. In some embodiments, the cell is a human cell. In some embodiments, the cell is a cancer cell. In some embodiments, the cell is a virus infected cell. In some embodiments, the cell is extracted from a subject in need thereof. In some embodiments, a subject in need thereof is a subject that has cancer. In some embodiments, a subject in need thereof is a subject suspected of having cancer. In some embodiments, a subject in need thereof is a subject that has a microorganism infection. In some embodiments, a subject in need thereof is a subject suspected of having a microorganism infection. In some embodiments, a subject in need thereof is a subject that has cancer or a microorganism infection. In some embodiments, a subject in need thereof is a subject suspected of having cancer or a microorganism infection.

In some embodiments, treating growth of a cell is treating a microorganism infection. In some embodiments, treating is killing. In some embodiments, treating is slowing, retarding or arresting growth. Each possibility represents a separate embodiment of the invention. In some embodiments, treating is a treatment that would treat a subject comprising the cell. In some embodiments, the cell is a cell of a microorganism and treating growth of the cell comprises treating a subject infected with the microorganism. In some embodiments, the cell is a cancer cell and treating growth of the cell comprises treating a subject with the cancer.

In some embodiments, the sample is a water sample. In some embodiments, the water sample is suspected of having a microorganism growing within it. In some embodiments, the water sample is a drinking water sample. In some embodiments, the water sample is an irrigation sample. In some embodiments, the drinking water sample is from a water pipe. In some embodiments, the water sample is from a water cooler, or domestic water appliance. As used herein, the term "domestic water appliance" refers to any home appliance that uses water. Examples of domestic water appliances include, but are not limited to water coolers, water bubblers, refrigerators, freezers, ice makers, drink carbonators, coffer/espresso machines, and vacuums.

In some embodiments, the system is for measuring changes in the amount of biomaterial present in a compartment. As used herein, "biomaterial" refers to any biological material produced by an organism. In some embodiments, biomaterial comprises secretions, extracellular matrix, proteins, lipids, organelles, membranes, cells, portions thereof, and combinations thereof. In some embodiments, cellular material comprises secretions, extracellular matrix, proteins, lipids, organelles, membranes, cells, portions thereof, and combinations thereof. In some embodiments, biomaterial comprises viruses. In some embodiments, the biomaterial is a replicating virus and thus comprises virus infected cells.

In some embodiments, the method is for use in determining the identity of a cell in a sample. In some embodiments, the sample is administered into a plurality of diffraction gratings. In some embodiments, the plurality of diffraction gratings comprises at least two different selective growth media. In some embodiments, the at least two different selective growth media are in different diffraction gratings. It will be understood that the two media are not mixed in the same diffraction media. As used herein, the term "selective growth media" refers to media with a composition that only certain cells will grow on them. This can be due to the media have composition that promotes cell growth, enables cell growth, delays cell growth or arrests cell growth of certain cells. Selective media are well known in the art and may contain specific carbon sources or specific amino acids or other drugs or molecules that enable growth or inhibit growth by certain cells. In some embodiments, the selective media is selective for certain bacteria. In some embodiments, selective media is differential media. By using a sufficient number of different media with different characteristics the exact identity or a general identity of the cell that is in the sample can be determined. Examples of selective media include but are not limited to, eosin methylene blue containing media, YM media with low pH, Thayer-Martin medium, xylose lysine desoxyscholate containing media, MacConkey media, and X-gal containing media.

As used herein, the term "identifying" a cell refers to determining information about the cell. In some embodiments, identifying is determining the domain of the cell. In some embodiments, identifying is determining the kingdom of the cell. In some embodiments, identifying is determining the phylum of the cell. In some embodiments, identifying is determining the class of the cell. In some embodiments, identifying is determining the order of the cell. In some embodiments, identifying is determining the family of the cell. In some embodiments, identifying is determining the genus of the cell. In some embodiments, identifying is determining the species of the cell. In some embodiments, identifying is determining the cell type of the cell. In some embodiments identifying is determining the microorganism of origin of the cell. In some embodiments identifying is determining the antibody resistance of the cell.

It will be understood by one skilled in the art that the systems of the invention can be used to measure optical response. Optical response comprises, among other elements, changes in the optical depth of the compartments of the grating. When any biological organism capable of reproducing is put in the grating the system can measure its rate of reproduction. The system does this in real-time as there can be continuous monitoring of the compartments. Further, by employing light with a limited waveband and analyzing zero and non-zero diffraction orders, very small changes in the effective optical depth can be detected. Thus, the system of the invention can detect even secretions, alterations of the extracellular matrix, creation of or alterations in a biofilm or enhanced intracellular volume that occur before a cycle of replication and can thus detect normal replication faster than any method currently existing in the art.

As used herein, a "diffraction grating" refers to a two-dimensional (2D) or 1D array of compartments, where the compartments have a dimension larger than the wavelength of light that is produced by the light source. In some embodiments, the array of compartments is ordered. In some embodiments all the compartments are identical in dimension. In some embodiments, the array comprises an irregular distribution. It will be understood that the compartment shape can vary or be constant and that the compartment dimensions can vary or be constant so long as the compartments comprise a dimension larger than the wavelength of the light that is produced by the light source. Examples of shapes of the compartments include, but are not limited to, squares, rectangles, circles, ovals and semi-circles. The compartments may be flat or rounded on the bottom or bottomless. Further, the compartments can be perforated or solid. In some embodiments, the compartment is transparent. In some embodiments, the compartment has a dimension such that a cell can fit therein. In some embodiments, the dimension is a lateral dimension. In some embodiments, the compartment is configured to be of a size sufficient for a cell to fit therein.

In some embodiments, the diffraction grating comprises a lamellar phase grating. In some embodiments, the diffraction grating comprises a photonic lamellar grating. In some embodiments, the diffraction grating comprises an ordered grating grid. In some embodiments, the diffraction grating is composed from transparent material. In some embodiments, the diffraction grating is composed from reflective material. In some embodiments, the diffraction grating is silicon based. In some embodiments, the diffraction grating is made from an inorganic material. In some embodiments, the diffraction grating is made from an organic material. In some embodiments, the diffraction grating is made from an organic or inorganic material. In some embodiments, a first portion of the diffraction grating will have a higher refraction index than a second portion of the diffraction grating. In some embodiments, the diffraction grating is etched in a silicon wafer. In some embodiments, the grating comprises at least one compartment configured to hold cells. In some embodiments, the grating comprises an array of compartments. In some embodiments, the grating is one-dimensional. In some embodiments, the grating is two-dimensional. In some embodiments, the grating is a linear grating. In some embodiments, the linear grating comprises at least 10, 50, 100, 200, 300, 400, 500, 600, 700, 800, 900 or 1000 compartments per mm, aligned linearly. Each possibility represents a separate embodiment of the invention. In some embodiments, the grating comprises up to 10000, 20000, 30000, 40000, 50000, 60000, 70000, 80000, 90000, 1000000, 200000, 300000, 400000, or 500000 compartments per square millimeters ($mm^2$). In some embodiments, the grating comprises up to 100,000 compartments per $mm^2$.

The terms "compartments" and "wells" are used herein interchangeably and refer to an enclosed part of the diffraction grating of sufficient size to hold the biological material that is being analyzed. It will be understood that the well must have sufficient space to allow cellular replication to proceed unimpeded by the dimensions of the well. In some embodiments, the well is configured to have sufficient room for the volume of media needed to provide non-limiting amounts of nutrients to the biological material. In some embodiments, the well is open to a reservoir of media. In some embodiments, the compartment has lateral dimensions such that at least 1, 5, 10, 15, 20, 25, 30, 35, 40, 45, or 50 cells can fit therein. Each possibility represents a separate embodiment of the invention. In some embodiments, the compartment has lateral dimensions such that at most 75, 100, 125, 150, 175, 200, 300, 400, 500, 600, 700, 800, 900, 1000, 5000, 10000, or 50000 cells can fit therein. Each possibility represents a separate embodiment of the invention. In some embodiments, the cells must adhere to the surface of the wells. In such embodiments, there must be sufficient room for the cells to adhere and reproduce without the space becoming limiting in the time frame of the detection. In some embodiments, well is configured to hold cells from at least one replication cycle. In some embodiments, the well is configured to hold cells from up to 2, 3, 4, 5, 6, 7, 8, 9, or 10 replication cycles. Each possibility represents a separate embodiment of the invention. In some embodiments, the well is configured to hold cells from up to 10 replication cycles. In some embodiments, the compartment has lateral dimensions such that at least 1, 10, 20, 30, 40, 50, 60, 70, 80, 90, 100 or 1000 cells can adhere therein. Each possibility represents a separate embodiment of the invention. In some embodiments, the well is configured to hold at least 1, 10, 20, 30, 40, 50, 60, 70, 80, 90, 100 or 1000 cells within half its volume. Each possibility represents a separate embodiment of the invention. In some embodiments, the well is configured to hold about 100 cells in half its volume.

It will be understood by one skilled in the art that the system and methods of the invention are for use with a diffraction grating that has a dimension greater than the wavelength of light being used. Though biomaterial may be smaller than the wavelength of light being used, for example, a virus, though much smaller than the wavelength of light still requires a host cell with dimensions larger than the wavelength of light.

In some embodiments, the diffraction grating comprises cell growth media. In some embodiments, the cell growth media is provided to the diffraction grating. In some embodiments, the cell is added to the cell growth media in the diffraction grating. Cell growth media is well known in the art, and various medias exist for different cell types. Examples of cell growth media include, but are not limited to DMEM, RPMI, Muller-Hinton Broth, and LB medium. In some embodiments, the cell growth media is a liquid. In some embodiments, the cell growth media is not a solid.

In some embodiments, the surface of the grating and/or compartment is configured to promote cell adhesion. In some embodiments, the diffraction grating, and/or a compartment therein comprises isopropenyl-ester or propylmethcrylate surface functional groups. In some embodiments, the diffraction grating, and/or a compartment therein comprises isopropenyl-ester functional groups. In some embodiments, the diffraction grating, and/or a compartment therein comprises propylmethcrylate surface functional groups. In some embodiments, the diffraction grating, and/or a compartment therein comprises surface double bond oxygen functional groups. In some embodiments, the diffraction grating, and/or a compartment therein comprises surface alkyl functional groups. In some embodiments, the surface groups increase cell adhesion to the surface. In some embodiments, the surface groups increase cell adhesion to the surface relative to a surface that does not have the functional groups. In some embodiments, the surface groups increase cell adhesion by at least 1%, 5%, 10%, 20%, 30%, 40%, 50%, 60%, 70%, 80%, 90, 100%, 200%, 300%, 400%, 500%, 600%, 700%, 800%, 900%, 1000%, 2000%, 3000%, 4000%, 5000%, 7500%, or 10000%. Each possibility represents a separate embodiment of the invention. In some embodiments, the surface groups increase cell adhesion by at least 1000%. In some embodiments, the surface groups reduce settlement and colonization time by at least 1%, 5%, 10%, 20%, 20%, 40%, 50%, 60%, 70%, 80%, 90%, 95%, 97%, or 99%. Each possibility represents a separate embodiment of the invention. In some embodiments, the surface groups result in the subsequent cells' exponential growth phase faster than if there were no attractant. In some embodiments, the surface groups reduce the time until exponential growth by at least 1%, 5%, 10%, 20%, 20%, 40%, 50%, 60%, 70%, 80%, 90%, 95%, 97%, or 99%. Each possibility represents a separate embodiment of the invention. In some embodiments, the surface groups result in a reduction of the time needed to complete the analysis of the cells' growth. In some embodiments, the reduction in time is a reduction of at least 1%, 5%, 10%, 20%, 20%, 40%, 50%, 60%, 70%, 80%, 90%, 95%, 97%, or 99%. Each possibility represents a separate embodiment of the invention.

In some embodiments, the diffraction grating, and/or a compartment therein is coated with a cell attractant. In some embodiments, only the inside of a compartment is coated with a cell attractant. In some embodiments, only the bottom of a well where the cells are to adhere is coated with a cell attractant. As used herein, the term "cell attractant" refers to any molecule that will recruit the cell to the site of the attractant. In some embodiments, the cell attractant recruits the cell by chemotaxis. In some embodiments, the cell attractant is configured to diffuse from the surface of the well into the surrounding media, thereby creating a concentration gradient. In some embodiments, the concentration gradient recruits the cell by chemotaxis. In some embodiments, the cell attractant is sugar molecules. In some embodiments, the sugar is sucrose. In some embodiments, the cell attractant is adsorbed to a surface of the compartment. In some embodiments, the cell attractant is adhered to a surface of the compartment. In some embodiments, the cell attractant is semi-stably adsorbed to a surface of the compartment. In some embodiments, the cell attractant is semi-stably adhered to a surface of the compartment. In some embodiments the surface is the bottom of the compartment where the cells are to adhere. In some embodiments, the semi-stable attractant is stable enough to remain on the surface when the compartment is dry, but upon addition of a liquid, such as media, the attractant can diffuse into the media thereby creating a gradient. In some embodiments, the grating with the attractant is kept wet at all times.

In some embodiments, the cell attractant facilitates faster settlement and colonization of cells to the surface of the well than if there were no attractant. In some embodiments, the cell attractant improved inoculation efficiency. In some embodiments, the attractant increases inoculation efficiency by at least 1%, 5%, 10%, 20%, 30%, 40%, 50%, 60%, 70%, 80%, 90, 100%, 200%, 300%, 400%, 500%, 600%, 700%, 800%, 900%, 1000%, 2000%, 3000%, 4000%, 5000%, 7500%, or 10000%. Each possibility represents a separate embodiment of the invention. In some embodiments, the attractant reduces settlement and colonization time by at least 1%, 5%, 10%, 20%, 20%, 40%, 50%, 60%, 70%, 80%, 90%, 95%, 97%, or 99%. Each possibility represents a separate embodiment of the invention. In some embodiments, the attractant reduces settlement and colonization time by at least 90%. In some embodiments, the cell attractant results in the subsequent cells' exponential growth phase faster than if there were no attractant. In some embodiments, the attractant reduces the time until exponential growth by at least 1%, 5%, 10%, 20%, 20%, 40%, 50%, 60%, 70%, 80%, 90%, 95%, 97%, or 99%. Each possibility represents a separate embodiment of the invention. In some embodiments, the attractant reduces the time until exponential growth by at least 90%. In some embodiments, the cell attractant results in a reduction of the time needed to complete the analysis of the cells' growth. In some embodiments, the reduction in time is a reduction of at least 1%, 5%, 10%, 20%, 20%, 40%, 50%, 60%, 70%, 80%, 90%, 95%, 97%, or 99%. Each possibility represents a separate embodiment of the invention. In some embodiments, the reduction in time is a reduction of at least 90%.

As used herein, the term "limited waveband" refers to light with a limited range of wavelengths. In some embodiments, the light is of a wavelength between 400 and 2000 nm. In some embodiments, limited waveband light is monochromatic light. In some embodiments, the light source is a monochromatic light source. In some embodiments, the light source is configured to illuminate at a limited waveband of not more than 1, 2, 5, 10, 15, 20, 25, 30, 35, 40, 45, or 50 nm.

Each possibility represents a separate embodiment of the invention. In some embodiments, the light source is configured to illuminate at a limited waveband of not more than 50 nm. In some embodiments, the light source is configured to illuminate at a limited waveband of not more than 25 nm. In some embodiments, limited waveband light is light with a bandwidth of not greater than 2, 5, 10, 15, 20, 25, 30, 35, 40, 45, or 50 nm. Each possibility represents a separate embodiment of the invention. In some embodiments, limited waveband light is light with a bandwidth of not greater than 50 nm. In some embodiments, limited waveband light is light with a bandwidth of not greater than 20 nm. In some embodiments the light is about a specific wavelength. In some embodiments, the light is laser light. In some embodiments, the light source is a laser. In some embodiments, the light source is a laser and the light has a limited waveband of not more than 5 nm. In some embodiments, the light source is a laser, laser diode (LD) or a light emitting diode (LED). In some embodiments, the light source is a LD. In some embodiments, the light source is a LED. In some embodiments, the light source is a LED and the light has a limited waveband of not more than 25 nm.

In some embodiments, monochromatic light is light of only one color based on the wavelength of the light. In some embodiments, red light is light between the wavelengths of 740 and 625 nanometers (nm). In some embodiments, red light is light between the wavelengths of 740 and 620 nm. In some embodiments, orange light is light between the wavelengths of 625 and 590, 625 and 585, 620 and 590 or 620 and 595 nm. Each possibility represents a separate embodiment of the invention. In some embodiments, yellow light is light between the wavelengths of 590 and 565, 590 and 560, 585 and 565 or 585 and 560 nm. Each possibility represents a separate embodiment of the invention. In some embodiments, green light is light between the wavelengths of 565 and 520, 565 and 500, 560 and 520 or 560 and 500 nm. Each possibility represents a separate embodiment of the invention. In some embodiments, cyan light is light between the wavelengths of 520 and 500, 520 and 480, or 500 and 480 nm. Each possibility represents a separate embodiment of the invention. In some embodiments, blue light is light between the wavelengths of 500 and 450, 500 and 435, 490 and 450, 490 and 435, 480 and 450 or 480 and 435. Each possibility represents a separate embodiment of the invention. In some embodiments, violet light is light between the wavelengths of 450 and 400, 450 and 380, 435 and 400 or 435 and 380 nm. Each possibility represents a separate embodiment of the invention.

In some embodiments, the laser light is red light. In some embodiments, the laser emits light with a wavelength of about 650 nm. In some embodiments, the light is not white light. In some embodiments, the light is not broadband light. In some embodiments, the light source does not emit a range of wavelengths. In some embodiments, the light source does not emit a spectrum of light.

As used herein, the term "coherent light" refers to light where the phase difference between the waves is constant such that the waves do not interfere with each other over a given distance. Coherent light sources are well known in the art and include lasers and LEDs. In some embodiments, the light source produces coherent light. In some embodiments, the light that hits the diffraction grating is coherent light. In some embodiments, the light has a coherence length of at least 1, 2, 3, 4, 5, 6, 7, 8, 9, 10, 15, 20, or 25 cm. Each possibility represents a separate embodiment of the invention. In some embodiments, the light has a coherence length of at least 1 cm.

In some embodiments, the light source is not perpendicular to the grating. In some embodiments, the light source is not normal to the grating. In some embodiments, the light hits the grating at any angle between 45 and 90 degrees. In some embodiments, the light hits the grating at any angle between 45 and 89 degrees. Each possibility represents a separate embodiment of the invention. In some embodiments, the light does not hit the grating at 90 degrees. In some embodiments, the light beam is coherent. In some embodiments, the light beam is collimated. In some embodiments, the light beam is coherent or collimated. In some embodiments, the light beam is coherent and collimated. In some embodiments, the system of the invention comprises a filter for colimiting the light.

As used herein, the terms "optical detection system" and "detector" are used synonymously and refer to an apparatus that is configured to detect the light emitted from the light source. In some embodiments, the optical detection system is configured to detect the diffraction intensities from narrow-band light. In some embodiments, the optical detection system is configured to detect the diffraction intensities from monochromatic light. In some embodiments, the optical detection system is not configured to detect white light, broadband light, or polychromatic light. Each possibility represents a separate embodiment of the invention. In some embodiments, the detector is an active pixel sensor (APS). In some embodiments, the APS is a complementary metal-oxide semiconductor (CMOS) sensor. In some embodiments, the detector is a charge-coupled device (CCD) sensor. In some embodiments, the detector comprises a light to frequency converter. In some embodiments, the light to frequency converter is a TSL253R light to frequency converter. In some embodiments, the sensor is a Pi Camera. In some embodiments, the sensor is a Raspberry Pi Camera.

According to the invention, the optical detection system 130 is configured to detect zero and non-zero diffraction orders. In some embodiments, the optical detection system is configured to detect all non-zero diffraction orders. In some embodiments, the system is configured to detect at least first and second order diffraction. In some embodiments, the system is configured to detect at least zero, first and second order diffraction. In some embodiments, the system is configured to detect at least two non-zero order diffractions. In some embodiments, the system is configured to detect at least a pair of odd and even order diffractions. In some embodiments, the system is configured to detect at least a pair of adjacent odd and even order diffractions. In some embodiments, measuring two non-zero diffraction orders provide more data than measuring zero order diffraction. In some embodiments, measuring zero and two non-zero diffraction orders provide more data than measuring just zero order diffraction. In some embodiments, measuring two non-zero diffraction orders provide greater resolution than measuring zero order diffraction. In some embodiments, measuring two non-zero diffraction orders allow for real time monitoring of a single compartment.

In some embodiments, the signal processing unit 140 is adapted to analyze non-zero diffraction orders. In some embodiments, the signal processing unit is adapted to analyze zero order diffraction. In some embodiments, the signal processing unit is adapted to analyze zero and non-zero diffraction orders. In some embodiments, the signal processing unit is adapted to determine time-dependent changes in effective optical depth of a grating. In some embodiments, the signal processing unit is adapted to determine time-dependent changes in effective optical depth of the grating. In some embodiments, the signal processing unit is adapted to determine a rate of cell growth. In some embodiments, the signal processing unit is adapted to determine a rate of change of biomaterial.

In some embodiments, the system further comprises a housing 115. In some embodiments, the system is deposed within the housing. In some embodiments, at least the diffraction grating is deposed in the housing. In some embodiments, the system further comprises a fluid inlet 111. In some embodiments, the sample and/or cell is brought to the diffraction grating via the fluid inlet. The fluid inlet may be a pipe, tube, trough or any similar structure that can bring a fluid to the diffraction grating. In some embodiments the system further comprises a fluid outlet 112. In some embodiments, the diffraction grating is deposed between the fluid inlet and outlet. In some embodiments, the diffraction grating is deposed within a housing with a fluid inlet and outlet into and out of the housing respectively. In some embodiments, the other components of the system are also deposed in the housing. In some embodiments, the other components of the system are deposed outside the housing. In some embodiments, the light source 120 is outside the housing. In some embodiments, the light source is within the housing. In some embodiments, the optical detection system is outside the housing. In some embodiments, the optical detection system is inside the housing. In some embodiments, the signal processing unit is inside the housing. In some embodiments, the signal processing unit is outside the housing. In some embodiments, the system of the invention is configured to be deposited within a water using domestic appliance such that it can monitor cell growth within the appliance, or within the water inside the appliance. In some embodiments, the water using domestic appliance is a water dispenser. In some embodiments, the housing is deposed within a water dispenser. In some embodiments, at least the diffraction grating 110 is deposed within a water dispenser. It will be understood that bacterial growth for instance in household appliances can be detrimental. If those appliances contain or have passing through them drinking water bacterial growth can be dangerous. As such the system of the invention can be installed into an appliance to monitor bacterial growth. In some embodiments, the system or at least the diffraction grating is deposited within a water filter. In some embodiments, the system further comprises a signal to notify when cellular growth reaches a predetermined threshold. In some embodiments, the signal processing unit is adapted to produce an output that indicates when the level or bacterial growth and/or that indicates when bacterial growth has reached a threshold level. In this way the system, can give an output such as an alarm or light that tells a user that an appliance should be cleaned or served due to bacterial growth.

By another aspect, there is provided a computer program as defined by claim 10.

In some embodiments, the data processor further determines a change in an amount of a biomaterial within the diffraction gratings. In some embodiments, the time-dependence is indicative of the growth kinetics of the biomaterial. In some embodiments, the calculating comprises comparing adjacent odd and even orders of diffraction. In some embodiments, the calculating comprises comparing odd and even orders of diffraction. In some embodiments, the calculating comprises comparing at least two non-zero orders of diffraction. In some embodiments, the calculating comprises finding the difference in intensity of adjacent odd and even orders of diffraction. In some embodiments, the calculating comprises finding the difference in intensity of a pair of odd and even orders of diffraction. In some embodiments, the calculating comprises finding the difference in intensity of at least two non-zero orders of diffraction.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Java, Smalltalk, C++ or the like, and conventional procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), application-specific integrated circuits (ASIC) or programmable logic arrays (PLA) may execute the computer readable program instructions by utilizing state information of the computer readable program instructions to personalize the electronic circuitry, in order to perform aspects of the present invention.

These computer readable program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

In some embodiments, the systems and/or computer program products of the invention are for use in performing the methods of the invention.

In some embodiments, change in the difference in intensities between an intensity of an odd order and intensity of an even order correlate to a change in effective optical depth. In some embodiments, the intensity of the even order is subtracted from the intensity of the odd order. In some embodiments, the intensity of the odd order is subtracted from the intensity of the even order. In some embodiments, an increase in effective optical depth indicates an increase in the amount of cellular material in said at least one compartment. In some embodiments, the system is optimized and/or calibrated in order to correlate the deviations in diffraction orders to the relative growth of said microorganism. In some embodiments, determining changes in effective optical depth comprises calibration of the diffraction orders' intensity with media of known refraction index. Diffraction indices for commonly used media are well known in the art or can be arrived at empirically by performing the detection of the invention with wells with no cells. In some embodiments, at least one grating is a control grating. In some embodiments, the control grating does not include biomaterial or cellular material. In some embodiments, the control grating is used to calibrate the detector and/or the signal processing unit. In some embodiments, a control standard is predetermined before performing the methods of the invention. In some embodiments, a result from a control grating is already provided such that they can be used for calibration without performing the control in parallel with the test well. In some embodiments, determining changes in effective optical depth comprises calibration of the detector by detecting diffraction orders' intensity obtained from illuminating a diffraction grating absent the at least one cell or sample. In some embodiments, a control diffraction grating is a grating that has not be inoculated with a cell. In some embodiments, a control diffraction grating is a grating that has only cell growth media. In some embodiments, the system comprises results from a control grating. In some embodiments, the signal processing unit comprises results from a control grating or a control experiment. In some embodiments, the system and/or the signal processing unit is preloaded with results from a control or a control experiment.

In some embodiments, the inoculating is with at least one cell. In some embodiments, the inoculating is with at least 1, 10, 100, 200, 300, 400, or 500 cells. Each possibility represents a separate embodiment of the invention. In some embodiments, the inoculating is with at least 400 cells. In some embodiments, the inoculation is with a solution comprising at least $10^1$, $10^2$, $10^3$, $10^4$, $10^5$, $10^6$, $10^7$ or $10^8$ cells/ml. Each possibility represents a separate embodiment of the invention. In some embodiments, the inoculating is with a solution comprising at least $10^3$ cells/ml. In some embodiments, the solution comprises about $10^3$ cells per ml. In some embodiments, the solution comprises about $10^3$ cells per ml and about 0.4 ml is inoculated.

In some embodiments, the inoculating results in at least 3 cells per square millimeter of diffraction grating. In some embodiments, the inoculating results in at least 1, 2, 3, 4, 5, 10, 15, 20, 25, or 30 cells per square millimeter of diffraction grating. Each possibility represents a separate embodiment of the invention.

In some embodiments, the methods of the invention can be performed in less than 10 minutes, 30 minutes, 45 minutes, 1 hour, 1.5 hours, 2 hours, 2.5 hours 3 hours, 4 hours, 5 hours, 6 hours, 7 hours, 8 hours, 9 hours, 10 hours, 15 hours, 20 hours, or 24 hours. Each possibility represents a separate embodiment of the invention. In some embodiments, the methods of the invention can be performed in about 30 minutes. In some embodiments, the methods of the invention can be performed in about 90 minutes. In some embodiments, the methods of the invention can be performed in less than 3 hours. In some embodiments, the methods of the invention can be performed in less than 4 hours. In some embodiments, the methods of the invention can be performed in less than 6 hours. In some embodiments, determining a change in amount of cellular material occurs in less than 10 minutes, 30 minutes, 45 minutes, 1 hour, 2 hours, 3 hours, 4 hours, 5 hours, 6 hours, 7 hours, 8 hours, 9 hours, 10 hours, 15 hours, 20 hours, or 24 hours. Each possibility represents a separate embodiment of the invention. In some embodiments, determining a change in amount of cellular material occurs in less than 3 hours. In some embodiments, determining a change in amount of cellular material occurs in about 30 minutes. In some embodiments, determining a cell replication rate occurs in less than 10 minutes, 30 minutes, 45 minutes, 1 hour, 1.5 hours, 2 hours, 2.5 hours 3 hours, 4 hours, 5 hours, 6 hours, 7 hours, 8 hours, 9 hours, 10 hours, 15 hours, 20 hours, or 24 hours. Each possibility represents a separate embodiment of the invention. In some embodiments, determining a cell replication rate occurs in less than 90 minutes. In some embodiments, determining a cell replication rate occurs in less than 3 hours. In some embodiments, determining a cell replication rate occurs in about 30 minutes. It will be understood that the amount of time the methods of the invention require will be dependent on the rate of replication and/or growth of the biological material in the grating as well as the initial starting about of material or cells, thus faster replicating/growing biologicals as well as greater starting number and/or amount will be able to be analyzed faster.

In some embodiments, the methods of the invention can be performed, determining a change in amount of cellular material occurs, or determining a cell replication rate, within between 30 minutes and 3 hours, 30 minutes and 4 hours, 30 minutes and 6 hours, 30 minutes and 8 hours, 30 minutes and 10 hours, 30 minutes and 12 hours, 20 minutes and 24 hours, 1 hour and 3 hours, 1 hour and 4 hours, 1 hour and 6 hours, 1 hour and 8 hours, 1 hour and 10 hours, 1 hour and 12 hours, or 1 hour and 24 hours. Each possibility represents a separate embodiment of the invention. In some embodiments, the methods of the invention can be performed, determining a change in amount of cellular material occurs, or determining a cell replication rate occurs within between 30 minutes and 3 hours. In some embodiments, the methods of the invention can be performed, determining a change in amount of cellular material occurs, or determining a cell replication rate, in at least 90 minutes. In some embodiments, the methods of the invention can be performed, determining a change in amount of cellular material occurs, or determining a cell replication rate, in not more than 90 minutes.

In some embodiments, the inoculation is with at least 1, 3, 5, 10, 15, 20, 30, 40, 50, 100, 200, 300, 400, or 500 cells. Each possibility represents a separate embodiment of the invention. In some embodiments, the inoculation is with at least 10 cells. In some embodiments, the inoculation is with at least 3 cells. In some embodiments, the inoculation is with at least 3 cells per square millimeter of diffraction grating. In some embodiments, the inoculation is with at least 1, 3, 5, 10, 15, 20, 30, 40, 50, 100, 200, 300, 400 or 500 cells/ml in an inoculation solution. Each possibility represents a separate embodiment of the invention. In some embodiments, the inoculation is with at least 10 cells/ml in an inoculation solution. In some embodiments, the inoculation is with about 300 cells.

In some embodiments, the inoculation is with at least 3 cells per square millimeter of diffraction grating and the method of the invention is performed in not more than 90 minutes. In some embodiments, the diffraction grating comprises a cell attractant. In some embodiments, the cell attractant facilitates performance of the method within 90 minutes with as few as 3 cells per square millimeter of diffraction grating.

In some embodiments, the methods of the invention are performed with at least 90, 91, 92, 93, 94, 95, 96, 97, 98, 99 or 100% accuracy. Each possibility represents a separate embodiment of the invention. In some embodiments, the methods of the invention are performed with at least 95% accuracy. In some embodiments, detection of cellular replication is performed with 95% accuracy. In some embodiments, detection of the presence of a cell in a sample is performed with 95% accuracy. As used herein, the term "accuracy" refers to the correct identification of changes in optical depth in the diffraction grating. Thus, an accuracy of 95% would mean that if 100 grating were measured for biomaterial growth and/or replication the system would correctly identify that growth or replication at least 95 times. In some embodiments, the accuracy is in positive detection of growth and/or replication. In some embodiments, the accuracy is in not detecting growth and/or replication in a diffraction grating in which it did not occur. In some embodiments, accuracy comprises the false positive rate. In some embodiments, accuracy comprises the false negative rate. In some embodiments, the false negative rate is less than 10, 9, 8, 7, 6, 5, 4, 3, 2, 1 or 0%. Each possibility represents a separate embodiment of the invention. In some embodiments, the false negative rate is less than 5%. In some embodiments, the false negative rate is less than 1%. In some embodiments, the false positive rate is less than 10, 9, 8, 7, 6, 5, 4, 3, 2, 1 or 0%. Each possibility represents a separate embodiment of the invention. In some embodiments, the false positive rate is less than 5%. In some embodiments, the false positive rate is less than 4%. In some embodiments, the system of the invention comprises the above recited accuracy, false positive rate and/or false negative rate.

In some embodiments, the infection is a bacterial infection. In some embodiments, the infection is a fungal infection. In some embodiments the infection is a yeast infection. In some embodiments, the infection is a bacterial, fungal or yeast infection. In some embodiments, the microorganism originated from an infection selected from a urinary infection, a vaginal infection, a respiratory infection, a cerebral infection, a bowel infection, a blood infection, a liver infection, a cardiac infection, and a cerebrospinal fluid infection.

In some embodiments, the subject is naive to treatment for the infection. In some embodiments, the subject has undergone treatment for the infection. In some embodiments, the treatment was ineffective. In some embodiments, the treatment was effective. In some embodiments, the subject has not yet received treatment, has undergone ineffective treatment, or has undergone effective treatment. In some embodiments, the subject is suspected of having an infection. In some embodiments, the methods of the invention are used for determining an infection. In some embodiments, the methods of the invention are used for monitoring infection progression. In some embodiments, the methods of the invention are used for monitoring infection remission. In some embodiments, the methods of the invention are used for monitoring continuation of an infection-free state in the subject. In some embodiments, the methods of the invention are used for selecting a therapeutic agent for the infection.

In some embodiments, the methods of the invention further comprise adding a test agent to the grating after inoculation, wherein the test agent has the potential to alter the optical properties of the at least one cell. In some embodiments, altering the optical properties of a cell comprises at least one of altering replication, altering the cell cycle, altering cell secretion, altering cellular metabolism, altering cell viability, and altering cell survival. In some embodiments, an optical property of a cell is indicative of at least one of cell viability, cell replication, cell motility, cellular metabolism, protein production, lipid production, volume expansion and secretion. In some embodiments, an optical property of a cell is at least one of cell volume, cell mass and cell density.

In some embodiments, the at least one therapeutic agent and or test agent is selected from an antibiotic, a chemotherapeutic, a radioisotope, a fungicide, an antiviral agent, a biostatic agent, an antibody, an immune cell and a virus. In some embodiments, the at least one therapeutic agent or test agent is an antibiotic. In some embodiments, different therapeutic agents are applied to different gratings. In some embodiments, different concentrations of a therapeutic agent are applied to different gratings. In some embodiments, the methods of the invention are used to determine a minimum inhibitory concentration (MIC) of a therapeutic agent.

As used herein, the term "about" when combined with a value refers to plus and minus 10% of the reference value. For example, a length of about 1000 nanometers (nm) refers to a length of 1000 nm+- 100 nm.

It is noted that as used herein and in the appended claims, the singular forms "a," "an," and "the" include plural referents unless the context clearly dictates otherwise. Thus, for example, reference to "a polynucleotide" includes a plurality of such polynucleotides and reference to "the polypeptide" includes reference to one or more polypeptides and equivalents thereof known to those skilled in the art, and so forth. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

In those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the invention are specifically embraced by the present invention and are disclosed herein just as if each and every combination was individually and explicitly disclosed. In addition, all sub-combinations of the various embodiments and elements thereof are also specifically embraced by the present invention and are disclosed herein just as if each and every such sub-combination was individually and explicitly disclosed herein.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

Various embodiments and aspects of the present invention as delineated hereinabove and as claimed in the claims section below find experimental support in the following examples.

## EXAMPLES

Generally, the nomenclature used herein, and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Strategies for Protein Purification and Characterization - A Laboratory Course Manual" CSHL Press (1996). Other general references are provided throughout this document.

### Optical Principles

### Example 1: Grating preparation and setup

The grating grids were fabricated from Silicon wafers and underwent thermal oxidation to produce a stable external layer of silicon with a thickness of about 100 nm. The gratings were cleaned with $H_2SO_4/H_2O_2$ solution and their surface were further modified to include isopropenyl-esters or propylmethcrylate as surface functional groups. Before use, the gratings were wetted with ethanol and placed within the device detection channels.

In a standard set-up illustrated in **Figure 1**, system 100 may include a silicon grating grid element 110 is placed in a temperature-controlled basin, segmented to 8 individual channels by elastomeric dividers. The channels are sealed by pressing a thin glass to the elastomers, effectively creating 20 mm long, 2 mm by 3 mm channels. A laser module 120 is directed toward the spot in each channel where the grating elements were placed. The light non-zero diffraction orders that reflect from the grating elements are acquired by a light sensor array 130 and the intensity distribution of the diffraction orders is recorded. The flow channels allow for easy addition of solutions (e.g. bacteria suspension, growth media, antibiotics, etc ...) as they pass above the grating elements, and also allow for easy disposal of samples after each analysis is complete.

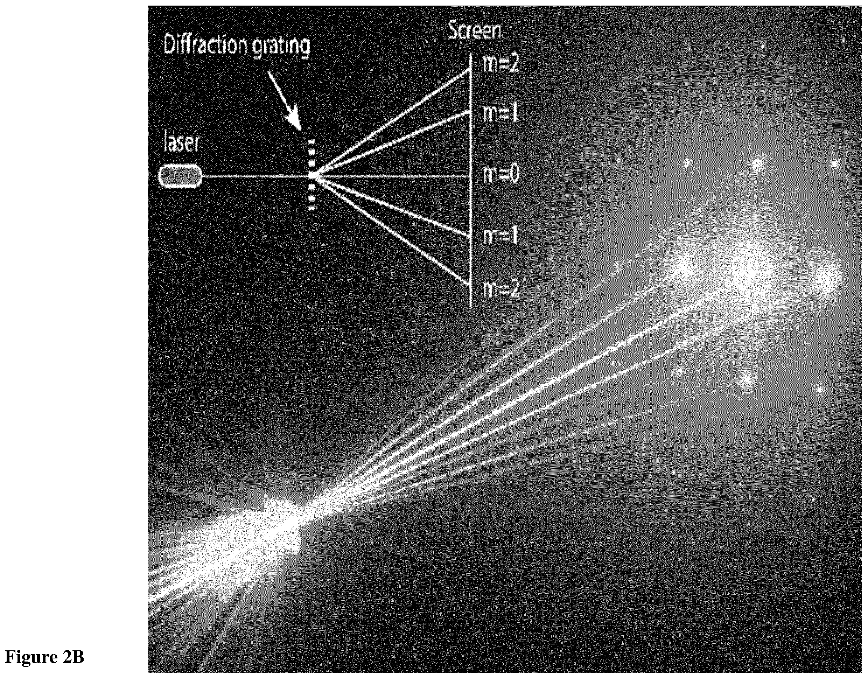

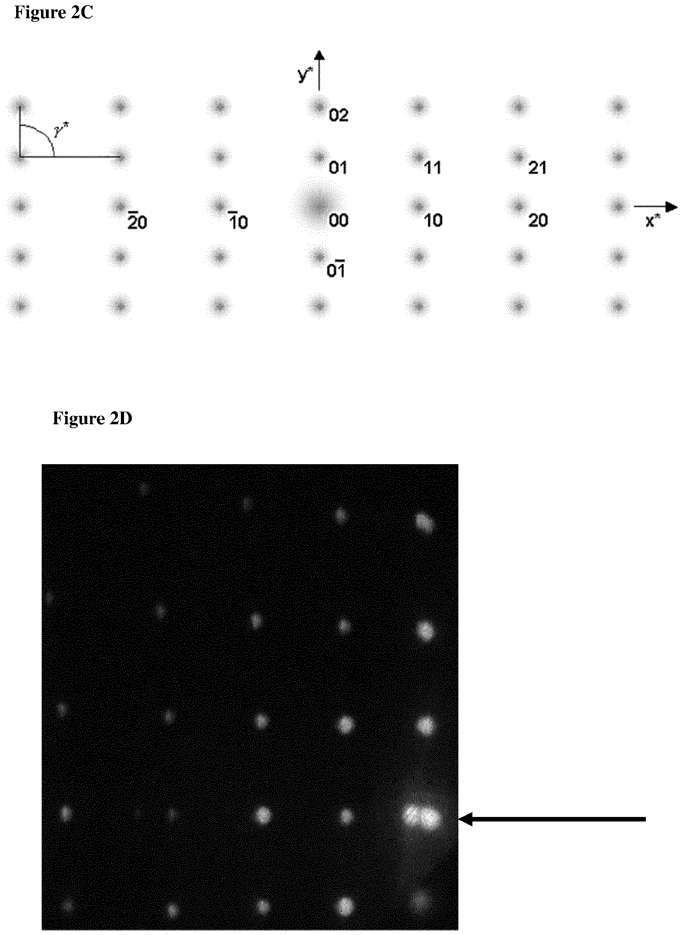

Light passing through an optical grating produces a diffraction pattern. **Figure 2A** shows a schematic of the phase grating and **Figure 2B** shows laser light passing through such a grating and creating diffraction orders. The refraction indexes of the two kinds of materials are noted by **$n_1$** and **$n_2$**, respectively. **d** and **b** are the grating dimensions, **w** is the grating depth and **m** is the diffraction order. **L** is the total width of all the gratings. The angle of diffraction depends on the distance between gratings and is generally expressed as $D \cdot sin\theta = n\lambda$. Thus, changes to the intra-grating dimensions will change the spatial location of all diffraction orders. When two diffraction gratings are superimposed perpendicularly (as in the 2D grating grid of the invention), they form a two-dimensional periodic array of apertures. The observed diffraction pattern is neither the sum nor the product of the original patterns of the individual gratings, but the separate patterns are repeated to form a two-dimensional array. Diffraction pattern of a two-dimensional arrays is analogous to the reciprocal lattice of the array and can be labelled (indexed) as shown in **Figure 2C**. Due to symmetry of both grating axes, the quarters of the diffraction distribution are identical reflections of each other. **Figure 2D** demonstrate such a diffraction pattern quarter of the reflection made by 650 nm laser beam from a silicon grating grid. As the laser is not accurately perpendicular to the grating, the direct reflection is a little offset from the zero-order diffraction (arrow).

The light intensity at diffraction angle θ for each order m, is distributed as:

Equation 1:

$$I_{(\theta)} = L^2 \cdot \sum_{-\infty}^{\infty} sinc^2\left(\frac{m}{2}\right) \cdot cos^2\left(\frac{\pi w(n_1 - n_2)}{\lambda} + \frac{m\pi}{2}\right) \cdot sinc^2\left(L\left(\frac{sin\theta}{\lambda} - \frac{m}{d}\right)\right)$$

For each diffraction order m, intensity values reach their maxima depending on the grating optical depth $w(n_1\text{-}n_2)$:

Equation 2:

$$\frac{\pi w(n_1 - n_2)}{\lambda} = \left(k + \frac{1}{2}\right)\pi, \quad k = 0, \pm 1, \pm 2, \ldots .$$

Due to the term $sinc^2$ (m/2), odd orders intensities shift in opposite direction to even orders intensities. Thus, $I_{(odd)}$ - $I_{(even)}$ will change when $w(n_1-n_2)$ does. This relationship can be termed in short as $\Delta I_m \propto \Delta n$. An example of a hypothetical refraction analysis is presented in **Figure 3A**. Measuring the intensities of two adjacent diffraction orders (odd 1st order and even 2nd order) one can plot their values as a function of solution refraction index. As expected from Eq. 1, the light intensity is counter-distributed as a function of the grating optical depth (here modulated by the solution refraction index). By plotting intensity shift ($\Delta I$, $I_{odd}-I_{even}$) the data can be summarized in a single parameter, wherein an increasing $\Delta I$ indicates increasing cellular biomass in the well.

Eq.1 should be viewed as sine series modulated by the cosine terms. The sinus phase number is denoted by g, that is the number of maxima or minima, $g_{max}$ or $g_{min}$, that precedes it.

For $g_{max, min}$ = 0; the transformation is y= Arccos(1-2x)/2
For $g_{max}$ = 1, 2, 3...; the transformation is y= Arcsin(1-2x)/2 +p*g
For $g_{min}$ = 1, 2, 3...; the transformation is y= Arccos(1-2x)/2 +p*g

Where p is a correction factor related to the specifics of the gratings manufacturing variations and is determined experimentally. For the grating of the example given in **Figure 3A** it was determined that p= -0.5. **Figure 3B,** shows the data from **Figure 3A** after applying this transformation.

## Example 2: Overcoming the limit of detection

The limit of detection (LOD) refers to the minimal concentration of bacteria in a sampled suspension, that can be positively detected within the device of the invention. **Figure 4A** depicts a grating with no bacteria at all. The dots present the actual measurement values, while the dark line represents the moving average. With an initial bacteria concentration of $10^5$/ml (**Fig. 4B**), the plot contains two segments: a 60 min lag phase, followed by an initial rising growth phase. If the initial bacteria concentration starts at $10^7$/ml (**Fig 4C**), the growth phase begins immediately without delay.

To improve the LOD and reduce the lag phase, an improved surface treatment that allows the semi-stable adsorption of sucrose molecules to the gratings was developed. The semi-stable sucrose deposit can sustain a concentration gradient in the solution above the grating that attracts bacteria cells through active chemotaxis. This was achieved by increasing the number of potential hydrogen bonds between the sugar side groups and the surface, through the addition of double bond oxygen molecules to the surface. The surface was then loaded by contact with a 2% sucrose solution in pH 8.

In applying the chemotaxis approach, lower bacteria starting concentration can be used to colonize the grating, both improving the LOD and truncating the lag phase. **Figures 5A-C** show plots taken from sucrose-treated gratings with initial bacteria concentrations of $10^3$/ml, $10^4$/ml and $10^5$/ml (**Fig. 5A**, **5B** and **5C**, respectively). In all these incidents, the growth phase begins promptly, without lagging effect and progress in a similar fashion. Thus, the use of sucrose treatment greatly enhances the speed with which the AST can be performed by eliminating the lag phase.

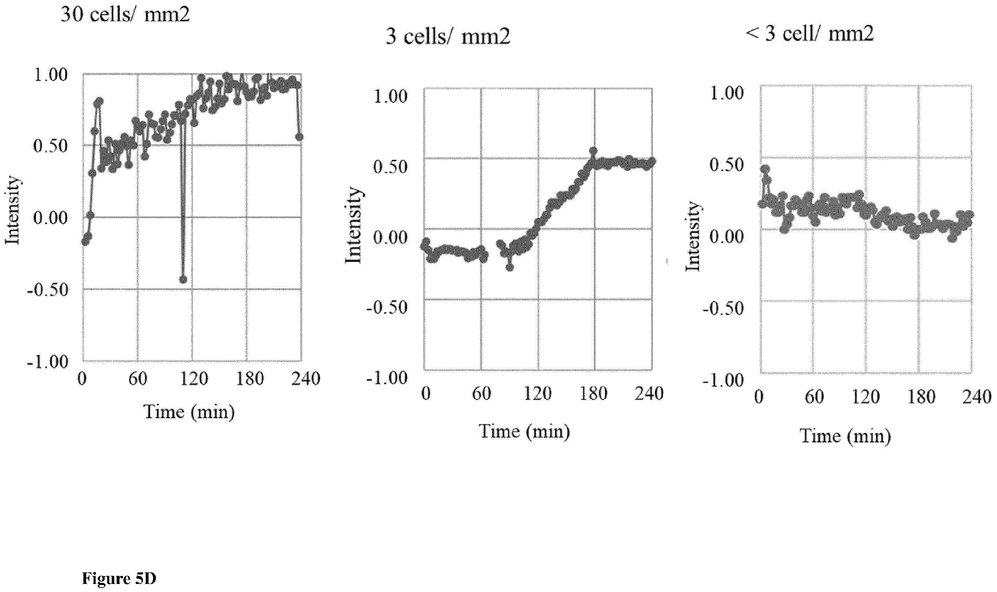

In order to determine the practical limit of detection as a number of cells per square millimeter of grating, 0.3 ml of diluted bacteria suspension was flowed onto a diffraction grating. Three concentrations of cells were tested, 30 cells per mm$^2$, 3 cells per mm$^2$ and less than 3 cells per mm$^2$ (**Fig. 5D**). Following the bacteria seeding stage (sample flow-through), the system received nutrient medium (Muller-Hinton) and data was collected every 3 minutes for 90 minutes. Detection of cell growth was possible even at only 3 cells per square millimeter of grating, indicating the extremely low limit of detection possible with this system in only an hour and a half.

## Example 3: Grating with bacterial cells

E. coli cells were inoculated into a well of the diffraction grating containing bacterial growth media and allowed to replicate freely. First and second order diffraction from this well, and a well containing only media, were monitored for 3 hours (**Fig. 6A** and **6B**, respectively). The increase in intensity shift ($\Delta I$) observed in the well containing bacteria (**Fig. 6C**) is indicative of the increase in optical depth over time due to the buildup of bacterial biomass in the grating. No change in $\Delta I$ was observed from the well lacking bacteria (**Fig. 6D**).

As the bacterial colonies are analyzed at sub-cell precision, changes can be observed even prior to the completion of a replication cycle. This aids completion of the analysis after very short incubation times. As explained above, the physical presence of a bacterium inside the grating contribute to the time-dependent change in grating optical depth and hence is expected to promote the difference between the intensity of *odd* diffraction orders and *even* ones. As the cells replicate, the growth in biomass, proportionally increase the optical depth, further widening the intensity gap between the diffraction orders.

In order to confirm that the system accurately predicts bacterial growth, samples of three bacterial strains (K. pneumonia, P. Mirabilis and E. Coli) were isolated from clinical urine samples from patients at the Laniado Medical Center in Netanya Israel. Bacteria colonies were inoculated in Muller-Hinton broth and the cell concentration in suspension was calculated by optical density analysis, followed by diluted to $10^4$ cell/ml in phosphate buffered water.

Each optical micro-fluidic channel in the system was prepared with a diffraction grating chip possessing chemo-attractant properties. Each channel in the system was an independent unit and was illuminated continuously with a dedicated laser source. 0.3 ml of bacterial suspension sample were added into each micro-fluidic channel, and incubated there for 30 min. Next, each micro-fluidic channel was rinsed with 0.5 ml of Enterobacteriaceae selective growth medium (McConkey), with or without antibiotics (1 $\mu$g Gentamycin). For K. Pneumonia, 24 samples were collected, of which 9 were grown in the presence of Gentamycin. For P. Mirabilis, 33 samples were collected, of which 12 were grown in the presence of Gentamycin. For E. Coli, 26 samples were collected, 15 of which were grown in the presence of Gentamycin. Laser diffraction reflected from each of the channels produced a diffraction image, that was recorded every 1 min, for 120 min. Image data was automatically analyzed to extract the intensity of each diffraction spot at every image. Experiments that suffered from documented mechanicals faults were excluded from further analysis.

For every experiment the trend in the relative diffraction intensity over time was calculated. Experiments that included strong deviations of >50% from trend line during the final 30 min, were excluded from the results. Shift, defined as the relative difference between the diffraction intensity at the beginning and end of measuring was used to determine if growth occurred. As all strains were antibiotic responsive, any well to which Gentamycin was added were expected to not have growth. For each bacteria, all gratings with expected growth were indeed measured to have growth within the 120 minutes of analysis. One false positive was also measured for each bacteria (Table 1). Thus, for measuring K. Pneumonia growth the system of the invention was 95.8% accurate, for P. Mirabilis the system was 96.9% accurate, for E. Coli the system was 96.15% accurate, which amounts to a total accuracy of over 96%.

Table 1: Summary of Growth Experiments

| Strain | N | False + | False - | Accuracy |
|--------|----|---------|---------|----------|
| K. Pneumonia | 24 | 1 | 0 | 95.80% |
| P. Mirabilis | 33 | 1 | 0 | 96.90% |
| E. Coli | 26 | 1 | 0 | 96.15% |
| Total | 83 | 3 | 0 | 96.39% |

## Example 4: Grating with bacterial cells and antibiotics

E. coli cells were inoculated into wells of the grating grid functionalized with semi-stable sucrose, as described herein, at a concentration of 10^4 cells/ml. Following the inoculation step, nutrient media was supplied while the diffractive optical output was monitored. The bacteria were grown for 60 min, before the device channels were supplemented with varying concentrations of G418 (Geneticin) antibiotic.

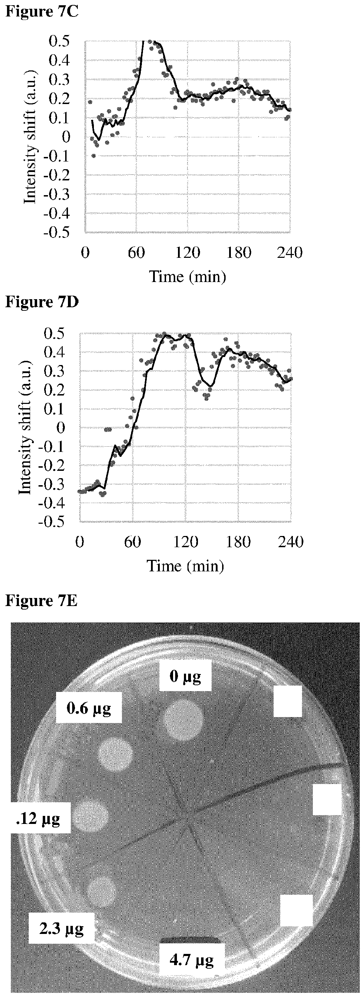

By monitoring the diffraction over time, a range for minimum inhibitory concentration (MIC) was established (**Fig. 7A-D**). A control well received no antibiotic and normal logarithmic growth was observed (**Fig. 7A**). Addition of 2.5 $\mu$g of G418 did not significantly inhibit bacterial growth (**Fig. 7B**). However, the addition of 5.0 $\mu$g (**Fig. 7C**) or 7.5 $\mu$g (**Fig. 7D**) completely inhibited bacterial growth. This would place the MIC at between 2.5 and 5.0 $\mu$g, which was the same as the range (2.3-4.7 $\mu$g) found by the classical method of serial antibiotic dilution (**Fig. 7E**).

## Claims

1. A system (100), the system comprising:

   a. a diffraction grating (110) comprising a compartment having lateral dimensions such that a cell can fit therein, optionally wherein said cell is a microorganism;
   b. a housing (115) and a fluid inlet (111) for bringing a sample comprising the cell and outlet (112) for extracting the sample comprising the cell and wherein said diffraction grating is deposed in the housing;
   c. a monochromatic light source (120) configured to produce a coherent and collimated light beam directed toward said diffraction grating;

d. an optical detection system (130) comprising a sensory array configured to detect zero and non-zero diffraction orders of said light beam from said diffraction grating; and

e. a signal processing unit (140) adapted to:

acquire from optical detection system zero and non-zero diffraction orders from the grating elements;

calculate differences in intensity of a pair of odd and even orders in the diffraction;

analyze the differences in the intensity to determine time-dependent changes in effective optical depth of said grating; and

determine therefrom a measure for growth of a cell in said grating.

2. The system of claim 1, wherein said diffraction grating (110)

a. is coated with a cell attractant, and optionally wherein said cell attractant is a sugar; or

b. contains a liquid suitable for cell survival and optionally wherein said cell attractant is configured to diffuse into said liquid thereby creating a concentration gradient;

optionally wherein said cell attractant is attached to said grating by hydrogen bonds to double-bond oxygen on said grating and optionally wherein said grating comprises an active group that comprises said double-bond oxygen.

3. The system of claim 1 or 2, wherein

a. said light source (120) is a laser, laser diode or a light emitting diode (LED), optionally wherein said laser is configured to illuminate at a waveband of at most 5 nm;

b. said optical detection system (130) comprises a light intensity detector

c. said optical detection system is configured to detect at least a first and second order diffraction of said light beam.

4. The system of any one of claims 1 to 3, further comprising a housing and a fluid inlet and outlet and wherein at least said diffraction grating is deposed in said housing, optionally wherein said system is deposed within a water dispenser.

5. A method for real-time detection of cell replication or growth, the method comprising:

a. inoculating at least one cell into a diffraction grating (110) of a system of any one of claims 1 to 4;

b. illuminating said diffraction grating (110) by a coherent and collimated light beam produced by a monochromatic light source (120) of a system (100) of any one of claims 1 to 4, said grating comprising said at least one cell with said light beam;

c. detecting zero and non-zero diffraction orders of said light beam from said illuminated diffraction grating;

d. calculating differences in intensity of a pair of odd and even orders in the diffraction, and analyzing the differences in the intensity over time to determine changes in effective optical depth of said diffraction grating comprising said at least one cell and therefrom determining a change in amount of cellular material in said diffraction grating comprising said at least one cell over time; thereby detecting cell replication or growth in real-time.

6. The method of claim 5, wherein said cell is from an infectious microorganism, is a cancer cell or is from a subject that has or is suspected of having a microorganism infection or cancer.

7. The method of any one of claim 5 to 6, wherein

a. said cell is from a sample provided by a subject in need thereof or is a water sample; or

b. said cellular material comprises any one of cells, cell secretions, extracellular matrix, portions thereof and combinations thereof.

8. The method of any one of claims 5 to 7, wherein

a. said processing of diffraction orders comprises processing at least two, non-zero, diffraction orders and wherein an increase in the difference between intensities of an odd order and an even order indicates an increase in effective optical depth and an increase in the amount of cellular material in said grating;

b. wherein said determining changes in effective optical depth comprises calibration of said detector by detecting diffraction orders' intensity obtained from illumination of said diffraction grating absent said at least one cell or sample; or

c. both.

9. The method of any one of claims 5 to 8, wherein

a. wherein said inoculating comprises a final concentration of cells within said diffraction grating of at least 3 cells per square millimeter of said diffraction grating;

b. said method further comprises adding a test agent to said grating after inoculation, wherein said test agent has the potential to alter at least one of the viability, replication, motility, metabolism, protein production, lipid production and secretion of said at least one cell and optionally wherein said at least one therapeutic or test agent is selected from the group consisting of an antibiotic, a chemotherapeutic, a radioisotope, a fungicide, a biostatic agent, an antibody, an immune cell and a virus; or

c. both.

10. A computer program product, comprising a non-transitory computer-readable storage medium having program code embodied thereon, the program code, when executed in a system (100) as defined in claim 1, causes the signal processing unit (140) to:

acquire over time from optical detection system (130) zero and non-zero diffraction orders of monochromatic light that reflect from a grating element;

calculate differences in intensity of a pair of odd and even orders in the diffraction; analyze the differences in the intensity to determine changes in effective optical depth of diffraction gratings that produced the zero and non-zero diffraction orders of monochromatic light; and

determine therefrom a measure for growth of a cell in said grating.

11. The computer program product of claim 10, wherein

a. said data processor calculates time-dependence of the differences in zero and non-zero diffraction orders and optionally determines a change in an amount of a biomaterial within said diffraction gratings.

**Patentansprüche**

1. System (100), das System umfassend:

a. ein Beugungsgitter (110), das ein Fach umfasst, das seitliche Abmessungen aufweist, so dass eine Zelle hineinpassen kann, optional wobei die Zelle ein Mikroorganismus ist;

b. ein Gehäuse (115) und einen Fluideinlass (111) zum Hinbringen einer Probe, die die Zelle umfasst, und einen -auslass (112) zum Extrahieren der Probe, die die Zelle umfasst, und wobei das Beugungsgitter in dem Gehäuse angeordnet ist;

c. eine monochromatische Lichtquelle (120), die ausgestaltet ist, einen kohärenten und gebündelten Lichtstrahl zu erzeugen, der auf das Beugungsgitter gerichtet ist;

d. ein optisches Erfassungssystem (130), das eine sensorische Anordnung umfasst, die ausgestaltet ist, Null- und Nicht-Null-Beugungsordnungen des Lichtstrahls von dem Beugungsgitter zu erfassen; und

e. eine Signalverarbeitungseinheit (140), angepasst zum:

Erhalten von dem optischen Erfassungssystem von Null- und Nicht-Null-Beugungsordnungen von den Gitterelementen;

Berechnen von Intensitätsunterschieden eines Paars von ungeraden und geraden Ordnungen in der Beugung;

Analysieren der Intensitätsunterschiede, um zeitabhängige Änderungen an einer effektiven optischen Tiefe des Gitters zu bestimmen; und

Bestimmen daraus eines Wachstumsmaßes einer Zelle in dem Gitter.

2. System nach Anspruch 1, wobei das Beugungsgitter (110)

a. mit einem Zell-Lockstoff beschichtet ist, und optional wobei der Zell-Lockstoff ein Zucker ist; oder
b. eine Flüssigkeit enthält, die für das Zellüberleben geeignet ist, und optional wobei der Zell-Lockstoff ausgestaltet ist, in die Flüssigkeit zu diffundieren, wodurch ein Konzentrationsgradient erzeugt wird;

optional wobei der Zell-Lockstoff an dem Gitter durch Wasserstoffbindungen befestigt ist, um Sauerstoff doppelt an das Gitter zu binden, und optional wobei das Gitter eine aktive Gruppe umfasst, die den doppelt gebundenen Sauerstoff umfasst.

3. System nach Anspruch 1 oder 2, wobei

a. die Lichtquelle (120) ein Laser, eine Laserdiode oder eine Leuchtdiode (LED) ist, optional wobei der Laser ausgestaltet ist, in einem Wellenbereich von höchstens 5 nm zu leuchten;
b. das optische Erfassungssystem (130) eine Lichtintensitätserfassungsvorrichtung umfasst;
c. das optische Erfassungssystem ausgestaltet ist, mindestens eine Beugung erster und zweiter Ordnung des Lichtstrahls zu erfassen.

4. System nach einem der Ansprüche 1 bis 3, ferner umfassend ein Gehäuse und einen Fluideinlass und -auslass, und wobei mindestens das Beugungsgitter in dem Gehäuse angeordnet ist, optional wobei das System innerhalb eines Wasserspenders angeordnet ist.

5. Verfahren zur Echtzeiterfassung von Zellreplikation oder -wachstum, das Verfahren umfassend:

a. Inokulieren mindestens einer Zelle in ein Beugungsgitter (110) eines Systems nach einem der Ansprüche 1 bis 4;
b. Beleuchten des Beugungsgitters (110) mit einem kohärenten und gebündelten Lichtstrahl, der durch eine monochromatische Lichtquelle (120) eines Systems (100) nach einem der Ansprüche 1 bis 4 erzeugt wird, wobei das Gitter die mindestens eine Zelle mit dem Lichtstrahl umfasst;
c. Erfassen von Null- und Nicht-Null-Beugungsordnungen des Lichtstrahls von dem beleuchteten Beugungsgitter;
d. Berechnen von Intensitätsunterschieden eines Paars von ungeraden und geraden Ordnungen in der Beugung und Analysieren der Intensitätsunterschiede im Zeitverlauf, um Änderungen an einer effektiven optischen Tiefe des Beugungsgitters zu bestimmen, das die mindestens eine Zelle umfasst, und Bestimmen daraus einer Änderung der Menge von zellularem Material in dem Beugungsgitter, das die mindestens eine Zelle umfasst, im Zeitverlauf;

wodurch Zellreplikation oder -wachstum in Echtzeit erfasst wird.

6. Verfahren nach Anspruch 5, wobei die Zelle aus einem infektiösen Mikroorganismus stammt, eine Krebszelle ist oder von einem Subjekt stammt, das an einer Mikroorganismusinfektion oder Krebs leidet oder mutmaßlich daran leidet.

7. Verfahren nach einem der Ansprüche 5 bis 6, wobei

a. die Zelle aus einer Probe ist, die von einem Subjekt mit entsprechendem Bedarf bereitgestellt wird, oder eine Wasserprobe ist; oder
b. das zellulare Material eines von Zellen, Zellsekretionen, extrazellularer Matrix, Teile davon und Kombinationen davon umfasst.

8. Verfahren nach einem der Ansprüche 5 bis 7, wobei

a. das Verarbeiten von Beugungsordnungen ein Verarbeiten von mindestens zwei, Nicht-Null-, Beugungsordnungen umfasst und wobei ein Anstieg des Unterschieds zwischen Intensitäten einer ungeraden Ordnung und einer geraden Ordnung auf einen Anstieg einer effektiven optischen Tiefe und einen Anstieg der Menge von zellularem Material in dem Gitter hinweist;
b. wobei das Bestimmen von Änderungen der effektiven optischen Tiefe ein Kalibrieren der Erfassungsvorrichtung durch Erfassen der Intensität von Beugungsordnungen umfasst, die durch ein Beleuchten des Beugungsgitters ohne die mindestens eine Zelle oder Probe erhalten wird; oder
c. beides.

9. Verfahren nach einem der Ansprüche 5 bis 8, wobei

a. das Inokulieren eine endgültige Konzentration von Zellen innerhalb des Beugungsgitters von mindestens 3 Zellen pro Quadratmillimeter des Beugungsgitters umfasst;
b. das Verfahren ferner ein Hinzufügen eines Testmittels zu dem Gitter nach dem Inokulieren umfasst, wobei das Testmittel das Potential aufweist, mindestens eines von Viabilität, Replikation, Motilität, Metabolismus, Proteinproduktion, Lipidproduktion und Sekretion der mindestens einen Zelle zu ändern, und optional wobei das mindestens eine Therapeutikum oder Testmittel aus der Gruppe bestehend aus einem Antibiotikum, einem Chemotherapeutikum, einem Radioisotop, einem Fungizid, einem biostatischen Mittel, einem Antikörper, einer Immunzelle und einem Virus ausgewählt ist; oder
c. beides.

10. Computerprogrammprodukt, das ein nicht-flüchtiges computerlesbares Speichermedium umfasst, auf dem ein Programmcode gespeichert ist, wobei der Programmcode, wenn er in einem System (100) nach Anspruch 1 ausgeführt wird, verursacht, dass die Signalverarbeitungseinheit (140):
von dem optischen Erfassungssystem (130) Null- und Nicht-Null-Beugungsordnungen von monochromatischem Licht, die von einem Gitterelement reflektiert werden, im Zeitverlauf erhält;

Intensitätsunterschiede eines Paars von ungeraden und geraden Ordnungen in der Beugung berechnet;
die Intensitätsunterschiede analysiert, um Änderungen an einer effektiven optischen Tiefe von Beugungsgittern zu bestimmen, die die Null- und Nicht-Null-Beugungsordnungen von monochromatischem Licht erzeugten; und
daraus ein Wachstumsmaß einer Zelle in dem Gitter bestimmt.

11. Computerprogrammprodukt nach Anspruch 10, wobei

a. der Datenprozessor eine Zeitabhängigkeit der Unterschiede von Null- und Nicht-Null-Beugungsordnungen berechnet und optional eine Änderung einer Menge eines Biomaterials innerhalb der Beugungsgitter bestimmt.

## Revendications

1. Système (100), le système comprenant :

a. un réseau de diffraction (110) comprenant un compartiment ayant des dimensions latérales telles qu'une cellule peut y entrer, facultativement dans lequel ladite cellule est un microorganisme ;
b. un logement (115) et une entrée de fluide (111) pour amener un échantillon comprenant la cellule et une sortie (112) pour extraire l'échantillon comprenant la cellule et dans lequel ledit réseau de diffraction est déposé dans le logement ;
c. une source de lumière monochromatique (120) configurée pour produire un faisceau lumineux cohérent et collimaté dirigé vers ledit réseau de diffraction ;
d. un système de détection optique (130) comprenant un réseau de capteurs configuré pour détecter des ordres de diffraction nuls et non nuls dudit faisceau lumineux provenant dudit réseau de diffraction ; et
e. une unité de traitement de signal (140) adaptée pour :

acquérir à partir du système de détection optique des ordres de diffraction nuls et non nuls à partir des éléments de réseau ;
calculer les différences d'intensité d'une paire d'ordres pair et impair dans la diffraction ;
analyser les différences d'intensité pour déterminer les changements dépendant du temps de la profondeur optique effective dudit réseau ; et
déterminer à partir de cela une mesure de croissance d'une cellule dans ledit réseau.

2. Système selon la revendication 1, dans lequel ledit réseau de diffraction (110)

a. est recouvert d'un agent attirant les cellules, et facultativement dans lequel ledit agent attirant les cellules est un sucre ; ou
b. contient un liquide approprié pour la survie cellulaire et facultativement dans lequel ledit agent attirant les cellules est configuré pour se diffuser dans ledit liquide, pour ainsi créer un gradient de concentration ;

facultativement dans lequel ledit agent attirant les cellules est fixé audit réseau par des liaisons hydrogène à un oxygène à double liaison sur ledit réseau et facultativement dans lequel ledit réseau comprend un groupe actif qui comprend ledit oxygène à double liaison.

3. Système selon la revendication 1 ou 2, dans lequel

   a. ladite source lumineuse (120) est un laser, une diode laser ou une diode électroluminescente (DEL), facultativement
   dans lequel ledit laser est configuré pour éclairer à une bande d'ondes d'au plus 5 nm ;
   b. ledit système de détection optique (130) comprend un détecteur d'intensité lumineuse
   c. ledit système de détection optique est configuré pour détecter au moins une diffraction de premier et de second ordre dudit faisceau lumineux.

4. Système selon l'une quelconque des revendications 1 à 3, comprenant en outre un logement et une entrée et sortie de fluide et dans lequel au moins ledit réseau de diffraction est déposé dans ledit logement, facultativement dans lequel ledit système est déposé dans un distributeur d'eau.

5. Procédé de détection en temps réel de la réplication ou de la croissance de cellules, le procédé comprenant :

   a. l'inoculation d'au moins une cellule dans un réseau de diffraction (110) d'un système selon l'une quelconque des revendications 1 à 4 ;
   b. l'éclairage dudit réseau de diffraction (110) par un faisceau lumineux cohérent et collimaté produit par une source de lumière monochromatique (120) d'un système (100) selon l'une quelconque des revendications 1 à 4, ledit réseau comprenant ladite au moins une cellule avec ledit faisceau lumineux ;
   c. la détection des ordres de diffraction nuls et non nuls dudit faisceau lumineux provenant dudit réseau de diffraction éclairé ;
   d. le calcul des différences d'intensité d'une paire d'ordres pair et impair dans la diffraction, et l'analyse des différences d'intensité au cours du temps pour déterminer les changements de profondeur optique effective dudit réseau de diffraction comprenant ladite au moins une cellule et la détermination à partir de cela d'un changement de quantité de matériel cellulaire dans ledit réseau de diffraction comprenant ladite au moins une cellule au cours du temps ;

   pour ainsi détecter la réplication ou la croissance de cellules en temps réel.

6. Procédé selon la revendication 5, dans lequel ladite cellule provient d'un microorganisme infectieux, est une cellule cancéreuse ou provient d'un sujet qui a ou est suspecté d'avoir une infection par un microorganisme ou un cancer.

7. Procédé selon l'une quelconque des revendications 5 à 6, dans lequel

   a. ladite cellule provient d'un échantillon fourni par un sujet qui en a besoin ou est un échantillon d'eau ; ou
   b. ledit matériel cellulaire comprend l'une quelconque parmi des cellules, des sécrétions cellulaires, une matrice extracellulaire, des parties de celles-ci et des combinaisons de celles-ci.

8. Procédé selon l'une quelconque des revendications 5 à 7, dans lequel

   a. ledit traitement des ordres de diffraction comprend le traitement d'au moins deux ordres de diffraction non nuls et dans lequel une augmentation de la différence entre les intensités d'un ordre impair et d'un ordre pair indique une augmentation de la profondeur optique effective et une augmentation de la quantité de matériel cellulaire dans ledit réseau ;
   b. dans lequel ladite détermination des changements de profondeur optique effective comprend l'étalonnage dudit détecteur en détectant l'intensité des ordres de diffraction obtenue à partir de l'éclairage dudit réseau de diffraction en l'absence de ladite au moins une cellule ou de l'échantillon ; ou
   c. les deux.

9. Procédé selon l'une quelconque des revendications 5 à 8, dans lequel

   a. dans lequel ladite inoculation comprend une concentration finale de cellules dans ledit réseau de diffraction d'au moins 3 cellules par millimètre carré dudit réseau de diffraction ;

b. ledit procédé comprend en outre l'ajout d'un agent de test audit réseau après l'inoculation, dans lequel ledit agent de test a le potentiel de modifier au moins l'un parmi la viabilité, la réplication, la motilité, le métabolisme, la production de protéines, la production de lipides et la sécrétion de ladite au moins une cellule et facultativement dans lequel ledit au moins un agent thérapeutique ou de test est sélectionné dans le groupe consistant en un antibiotique, un agent chimiothérapeutique, un radioisotope, un fongicide, un agent biostatique, un anticorps, une cellule immunitaire et un virus ; ou
c. les deux.

10. Produit de programme informatique, comprenant un support de stockage non transitoire lisible par ordinateur sur lequel est incorporé un code de programme, le code de programme, lorsqu'il est exécuté dans un système (100) tel que défini dans la revendication 1, amène l'unité de traitement de signal (140) à :

acquérir au cours du temps à partir d'un système de détection optique (130) des ordres de diffraction nuls et non nuls d'une lumière monochromatique qui se réfléchissent sur un élément de réseau ;
calculer les différences d'intensité d'une paire d'ordres pair et impair dans la diffraction ;
analyser les différences d'intensité pour déterminer les changements de profondeur optique effective des réseaux de diffraction qui ont produit les ordres de diffraction nuls et non nuls de lumière monochromatique ; et
déterminer à partir de cela une mesure de la croissance d'une cellule dans ledit réseau.

11. Produit de programme informatique selon la revendication 10, dans lequel

a. ledit processeur de données calcule la dépendance en fonction du temps des différences des ordres de diffraction nuls et non nuls et détermine facultativement un changement de quantité d'un biomatériau dans lesdits réseaux de diffraction.

Figure 1

**Figure 2A**

Figure 2B

**Figure 2C**

**Figure 2D**

**Figure 3A**

**Figure 3B**

**Figure 4A**

**Figure 4B**

**Figure 4C**

EP 3 921 626 B1

**Figure 5A**

**Figure 5B**

**Figure 5C**

30 cells/ mm2

3 cells/ mm2

< 3 cell/ mm2

Figure 5D

EP 3 921 626 B1

**Figure 6A**

**Figure 6B**

**Figure 6C**

**Figure 6D**

**Figure 7A**

**Figure 7B**

**Figure 7C**

**Figure 7D**

**Figure 7E**

## REFERENCES CITED IN THE DESCRIPTION

**Patent documents cited in the description**

- US 62627797
- US 20160061822 A1
- WO 2016142878 A1
- US 4666828 A
- US 4683202 A
- US 4801531 A
- US 5192659 A
- US 5272057 A

**Non-patent literature cited in the description**

- **SAMBROOK et al.** Molecular Cloning: A laboratory Manual. 1989
- Current Protocols in Molecular Biology. 1994, vol. I-III
- **AUSUBEL et al.** Current Protocols in Molecular Biology. John Wiley and Sons, 1989
- **PERBAL**. A Practical Guide to Molecular Cloning. John Wiley & Sons, 1988
- **WATSON et al.** Recombinant DNA. Scientific American Books
- Genome Analysis: A Laboratory Manual Series. Cold Spring Harbor Laboratory Press, 1998, vol. 1-4
- Cell Biology: A Laboratory Handbook. 1994, vol. I-III
- **FRESHNEY**. Culture of Animal Cells - A Manual of Basic Technique. Wiley-Liss, 1994
- Current Protocols in Immunology. 1994, vol. I-III
- Basic and Clinical Immunology. Appleton & Lange, 1994
- Strategies for Protein Purification and Characterization - A Laboratory Course Manual. CSHL Press, 1996